# EUROPEAN PATENT SPECIFICATION

(11) **EP 1 295 612 B1**
(45) Date of publication and mention of the grant of the patent: **06.12.2006**
(21) Application number: 00905308.3
(22) Date of filing: 24.02.2000
(51) Int. Cl.: A61L 2/18, C07K 14/435, A61F 2/16, A61L 2/00, A61L 12/10, A01N 59/00, C01B 11/04, C01B 11/06

(54) **USE OF A TREATING SOLUTION FOR INACTIVATING PRIONS**
VERWENDUNG EINER BEHANDLUNGLÖSUNG ZUR INAKTIVIERUNG VON PRIONEN
UTILISATION D'UNE SOLUTION DE TRAITEMENT POUR L'INACTIVATION DE PRIONS

(43) Date of publication of application: 26.03.2003
(73) Proprietor: Menicon Co., Ltd., Nagoya-shi, Aichi 460-0006 (JP)
(72) Inventor: SPITTLER, Joseph, F-67100 Strasbourg (FR); VILCOT, Pierre, F-60500 Chantilly (FR); TSUZUKI, Akira Menicon Co., Ltd., Kasugai-shi Aichi 487-0032 (JP)
(74) Representative: TBK-Patent
(86) International application number: PCT/JP2000/001053
(87) International publication number: WO 2001/062305

(56) References cited:
- GB-A- 1 484 972
- JP-A- 11 299 867
- US-A- 5 521 164
- DATABASE WPI Section Ch, Week 198417 Derwent Publications Ltd., London, GB; Class D25, AN 1984-103580 XP002231463 & JP 59 045399 A (TOYO CONTACT LENS CO LTD), 14 March 1984 (1984-03-14)
- DATABASE WPI Section Ch, Week 199234 Derwent Publications Ltd., London, GB; Class D21, AN 1992-279799 XP002231464 & JP 04 190214 A (HOYA CORP), 8 July 1992 (1992-07-08)
- DATABASE BIOSIS [Online] BIOSCIENCES INFORMATION SERVICE, PHILADELPHIA, PA, US; February 1999 (1999-02) DORMONT D: "Agents that cause transmissible subacute spongiform encephalopathies." Database accession no. PREV199900242352 XP002231462 & BIOMEDICINE & PHARMACOTHERAPY, vol. 53, no. 1, February 1999 (1999-02), pages 3-8, ISSN: 0753-3322

## Description

### TECHNICAL FIELD

The present invention relates to the use of a treating solution in a process for deactivating prions in safety, which is a pathogenic factor mixed in protein and protein-containing substance with maintaining physiological activity, specific quality and specific characteristic of protein and protein-containing substances, wherein the object to be treated is a contact lens.

### BACKGROUND ART

When a protein and a protein for food materials having physiology activity or special quality are prepared from tissue or body fluid of animals including human, there is a danger that contamination will arise by the pathogenic particle mixed in such tissue or body fluid. In relation to this, a pathogenic factor called prion has come under close scrutiny, recently. Although there are many still unknown points about this prion, it is called the transmissible protein which does not have DNA or RNA because the existence of nucleic acid is not proved. As a disease of human in which the prion is the pathogenic organ, Kuru, Creutzfeldt-Jakob disease, Gerstmann-Straussler syndrome and the like are conventionally known, and other than the disease of human Crapie in sheep and goats, bovine spongiform encephalopathy (mad cow disease) in cow, and the like are known. It is said that especially the above-mentioned Creutzfeldt-Jakob disease is spread to a patient by corneal grafting, endocranial grafting, administration of a growth hormone extracted from the human pituitary, the contaminated brain-waves electrode or the like.

It is known that the large amount of the above-mentioned transmissible pathogenic organism exists in brain and spinal cord of Kuru patients and that the similar transmissible pathogenic organism is detected in spleen, liver, lymph node, lung, spinal cord, renal, cornea, lens, cerebrospinal fluid and blood of Creutzfeldt-Jakob disease and Gerstmann-Straussler syndrome. However, since prion is not exhibit an immune response such as a generation of the antibody, the vaccine is not existed and it is very difficult to diagnose whether prion is infected or not.

The process for deactivating prion or the process for decaying the infecting ability of prion was conventionally studied variously. However, the prion has a high resistance to the conventional process for deactivating pathogenic microorganism and the process for decaying the infecting ability of pathogenic microorganism, for example, an irradiation, a boiling treatment, drying, and a treatment with chemical agent such as formalin, β-propiolactone, alcohol, iodine compound, acetone, potassium permanganate, hydrogen peroxide or ethylene oxide gas, and thus such process is not effective. Meanwhile, it is known that prion can be deactivated by the treatment with inorganic acidic or alkaline solution of high concentration at high temperature, the treatment with hypochlorous acid-alkaline solution, or the treatment with high-pressure steam (at 134°C, for 1 hour) (e.g. Japanese Unexamined Patent Publication No. 49611/1999). However, since the sodium salt of this hypochlorous acid and the like are the toxic substance causing dermatitis and respiratory distress when treatment on these severe conditions, especially a high-concentration hypochlorous acid are used, there is a possibility of vanishing the physiological activity and the specific quality of protein or degrading physical properties in irreversible.

For example, Japanese Unexamined Patent Publications No. 49611/1999 discloses the process for deactivating prion or the process for decaying the infecting ability thereof in which prion is treated with liquid alkenyl oxide having 2 to 4 carbon atoms. Although this process effectively deactivates prion and prevents protein from denaturing as compared with the case where the conventional gaseous ethylene oxide is used, the effect may not yet be satisfied. Therefore, it waits eagerly for development of the method which was further excellent in the deactivation effect of prion, and the prevention effect of preventing protein from denaturing.

GB-A-1 484 972 describes a method for sterilizing medical or surgical equipment using a solution containing hypochlorite ions in an amount to kill pathogenic micro-organisms.

JP-A-59 045 399 and JP-A-04 190 214 describe detergents for contact lenses comprising an aqueous solution of hypohalogenite and/or hypohalogenous acid.

In Biomedicine & Pharmacotherapy, vol. 53, no. 1, February 1999, pages 3-8, there is described a treatment of TSA/prions with either 1N NaOH or sodium hypochlorite (2% Cl) during 1 h at room temperature in order to inactivate prion.

The present invention is made in view of the above-mentioned background, and intends to provide a process for deactivating prions sufficiently and in safety with maintaining physiological activity, specific quality and specific characteristic of protein and protein-containing substances.

The present invention relates to the use of a treating solution, containing a hypohalogenite and/or hypohalogenous acid ion in concentration of at least 0.1 w/v % and less than 1 w/v %, for deactivating prions as defined in claim 1.

### BEST MODE FOR CARRYING OUT THE INVENTION

As a hypohalogenite contained in the above-mentioned treating solution, there are an alkaline metal salt and alkaline earth metal salt of hypohalogenous acid such as hypochlorous acid and hypobromous acid. Concretely, there are hypochlorites such as sodium hypochlorite, potassium hypochlorite and calcium hypochlorite; and hypobromites such as sodium hypobromite, potassium hypobromite and calcium hypobromite. Among them, sodium hypohalogenites such as sodium hypochlorite and sodium hypobromite are preferable for the reason that the deactivating effect on prion is high.

Examples of a hypohalogenous acid ion contained in the above-mentioned treating solution are a hypochlorous acid ion, a hypobromous acid ion and the like which are generated resulting from a reaction of the above-mentioned a hypohalogenite and an alkaline metal halide and an alkaline earth metal halide.

Concrete examples include the hypobromous acid ion generated from sodium hypochlorite and potassium bromide, the hypobromous acid ion generated from hypobromous acid calcium and potassium bromide and the like.

In addition, the hypohalogenous acid ion has the higher deactivating effect on prion than the above-mentioned hypohalogenite, so the hypohalogenous acid ion may be used especially suitable for the present invention.

The concentration of the hypohalogenite and/or hypohalogenous acid ion in the treating solution is at least 0.1 w/v %, preferably at least 0.3 w/v % in order to exhibit the effect of deactivation on prion, sufficiently. The concentration of the hypohalogenite and/or hypohalogenous acid ion in the treating solution is less than 1 w/v %, preferably at most 0.7 w/v % in order to increase the concentration of the residual hypohalogenite and/or hypohalogenous acid ion, to prevent a decrease of the safety, and to prevent physiological activity, specific quality and specific characteristic of protein and protein-containing substances from deteriorating, wherein the protein and the protein-containing substances may contain prion.

Unless the purpose of the present invention is harmed, the other components, for example, sodium carbonate and sodium hydroxide for pH adjustment can be added to the above-mentioned treating solution in addition to hypohalogenite and/or the hypohalogenous acid ion, appropriately.

Although there is especially no limitation in the preparation method of the treating solution, for example, the method shown below may be employed.

For example, in case of the treating solution containing a hypohalogenite, the hypohalogenite may be dissolved in distilled water and purified water so that the concentration of the hypohalogenite is within the above-mentioned specific range.

Additionally, in case of the treating solution containing a hypohalogenous acid ion, the following methods can be employed.
(a) The method that the aqueous solution of hypohalogenite is prepared and preserved in the suitable vessel, and used itself or by diluting the solution with distilled water or purified water so that the concentration of the hypohalogenous acid ion is within the above-mentioned specific range prior to use.
(b) The method for preparing a solution containing the hypohalogenous acid ion in the concentration within the above-mentioned specific range by dissolving a tablet, powder or a granule containing the hypohalogenite together with a tablet, powder or a granule containing the alkaline metal halide or alkaline earth metal halide in distilled water or purified water.
(c) The method using a tablet, powder or a granule of the compound, in which the tablet, powder or the granule can dissolve in distilled water or purified water to generate the hypohalogenous acid ion in the concentration within the above-mentioned specific range in the solution. Among them (b) and (c) is more practicable from a viewpoint of storage stability.

In the process, although the object to be treated is contacted to the above-mentioned treating solution containing hypohalogenite and/or hypohalogenous acid ion in the specific concentration, the contacting method is not particularly limited. For example, such method that the object to be treated is immersed in the treating solution may be employed.

The contacting time of the object to be treated with the treating solution is desirably at least 3 minutes, preferably at least 5 minutes, more preferably at least 10 minutes in order to exhibit the effect of deactivation on prion, sufficiently. The contacting time of the object to be treated with the treating solution is desirably at most 1 hour, preferably 50 minutes, more preferably at most 30 minutes in order to increase the concentration of residual hypohalogenite and/or hypohalogenous acid ion, and to prevent a decrease of the safety.

On contacting the object to be treated with the treating solution, pH of the treating solution is at least 10, preferably 13.

On contacting the object to be treated with the treating solution, it is desirable that the temperature of the treating solution is at least 0°C, preferably at least 10°C, and at most 60°C, preferably 50°C.

As the object to be treated according to the present invention, there is a contact lens.

As mentioned above, since a treating solution containing a hypohalogenite and/or hypohalogenous acid ion in the specific low concentration range and showing high safety is used, prion can be sufficiently deactivated with maintaining physiological activity, specific quality and specific characteristic of protein and protein-containing substances.

Then, the process for deactivating prions and the treating solution used therein are explained in more detail by the following Examples, but the present invention is not limited to the Examples.

### EXAMPLES 1 to 3 and COMPARATIVE EXAMPLE 1

First, four rigid gas permeable contact lenses (available from Menicon Co., Ltd., trade name: Menicon Z) contaminated with TSE (transmissible spongiform encephalopathy) prion was prepared.

Then, the above-mentioned contaminated contact lenses were immersed in the following each solution for a period described as follows.
- Example 1: 0.4 w/v % sodium hypochlorite solution
(pH: 12.3, solution temperature: room temperature)
Immersion time: for 5 minutes
- Example 2: 0.4 w/v % sodium hypochlorite solution
(pH: 12.3, solution temperature: room temperature)
Immersion time: for 30 minutes
- Example 3: the solution generating hypochlorous acid ion (in which hypochlorous acid ion is generated by adding 5 ml of 0.6 w/v % potassium bromide to 5 ml 0.4 w/v % sodium hypochlorite solution, pH: 11, solution temperature: room temperature)
Immersion time: for 5 minutes
- Comparative Example 1: 1.85 w/v % sodium hypochlorite solution
(pH: 12.3, solution temperature: room temperature)
Immersion time: for 1 hour

After immersion each contact lens was picked up from each solution and immersed in ultra pure water containing an antibiotics (solution temperature: 37°C) for 24 hours to extract prion. The test sample was prepared by adding a buffer containing glucose and hamster brain homogenate to the extract.

Each of the above-mentioned test samples was inoculated to brains of 12 hamsters (male) in amount of 0.5 mg. Expect for the above-mentioned test samples a test sample (the test sample in which the above-mentioned contaminated contact lens was not immersed in any solution used in Examples 1 to 3 and Comparative Example 1 but immersed in ultra pure water containing the antibiotics for 24 hours to extract prion and the buffer containing glucose and hamster brain homogenate were added to the extract) as positive control in amount of 0.5 mg was inoculated to brains of 12 hamsters (male), respectively.

During 9 months after the inoculation, an observation of the symptom specific to TSE (behavioral disorder, cerebellar ataxia and acroparalysis) and a confirmation of life or death were carried out for each hamster.

As the result, the all hamsters inoculated with the test sample of the positive control exhibited the symptom specific to TSE and thus died.

In addition, as the positive control, all 12 hamsters in the dilution of TSE prion by 100 times died within 85 days, all 12 hamsters in the dilution by 1,000 times died within 96 days, and 11 out of 12 hamsters in the dilution by 10,000 times died within 134 days. Furthermore, in the dilution by 100,000 times, the dilution by 1,000,000 times, the dilution by 10,000,000 times, all 12 hamsters exhibits the symptom specific to TSE within after 125 days, 142 days and 155 days, respectively.

On the contrary, when the contaminated contact lens was immersed in the treating solution described in Examples 1 to 3 and when immersed in the treating solution described in Comparative Example, in each case the symptom specific to TSE during 5 months was not observed and all hamsters were alive after 9 months.

From the result mentioned above, it is found that when the treating solution is used according to the present invention, the treating solution exhibits the deactivation effect on prion equally to that in case of immersion in 1.85 w/v % of sodium hypochlorite solution for 1 hour, even in immersion for only five minutes, and therefore is highly effective in preventing prion from infecting through contact lens.

### INDUSTRIAL APPLICABILITY

Since a treating solution containing a hypohalogenite and/or hypohalogenous acid ion in the specific low concentration range and showing high safety is used according to the present invention, prion can be sufficiently deactivated with maintaining physiological activity, specific quality and specific characteristic of protein and protein-containing substances.

## Claims

1. Use of a treating solution containing a hypohalogenite and/or hypohalogenous acid ion in concentration of at least 0.1 w/v % and less than 1 w/v % for deactivating prions, wherein the object to be treated by said treating solution is a contact lens.

2. Use according to claim 1, wherein the concentration of the hypohalogenite and/or hypohalogenous acid ion is 0.1 to 0.7 w/v %.

3. The use according to claim 1 or 2, wherein the contact lens is contacted with said treating solution.

4. The use of claim 1, wherein the hypohalogenite is hypochlorite or hypobromite.

5. The use of claim 1, wherein the hypohalogenous acid ion is hypochlorous acid ion or hypobromous acid ion.

6. The use of claim 1, wherein the object to be treated is contacted with the treating solution for 3 minutes to 1 hour.

7. The use of claim 1, wherein the object to be treated is contacted with the treating solution for 5 to 50 minutes.

8. The use of claim 1, wherein the object to be treated is contacted with the treating solution for 10 to 30 minutes.

9. The use of claim 1, wherein pH of the treating solution is 10 to 13.

10. The use of claim 1, wherein temperature of the treating solution is 0°C to 60°C.

## Patentansprüche

1. Verwendung einer Behandlungslösung enthaltend ein Hypohalogenit und/oder ein Ion einer hypohalogenigen Säure, mit einer Konzentration von mindestens als 0.1 w/v % und weniger als 1 w/v % zur Inaktivierung von Prionen, wobei der mit der Behandlungslösung zu behandelnde Gegenstand eine Kontaktlinse ist.

2. Verwendung nach Anspruch 1, wobei die Konzentration an Hypohalogenit und/oder eines Ions einer hypohalogenigen Säure 0.1 bis 0.7 w/v % ist.

3. Verwendung nach Anspruch 1 oder 2, wobei die Kontaktlinse mit der Behandlungslösung in Kontakt gebracht wird.

4. Verwendung nach Anspruch 1, wobei das Hypohalogenit entweder ein Hypochlorit oder ein Hypobromit ist.

5. Verwendung nach Anspruch 1, wobei das Ion der hypohalogenigen Säure entweder ein hypochloriges oder ein hypobromiges Säureion ist.

6. Verwendung nach Anspruch 1, wobei der zu behandelnde Gegenstand mit der Behandlungslösung 3 Minuten bis 1 Stunde in Kontakt gebracht wird.

7. Verwendung nach Anspruch 1, wobei der zu behandelnde Gegenstand mit der Behandlungslösung 5 Minuten bis 50 Minuten in Kontakt gebracht wird.

8. Verwendung nach Anspruch 1, wobei der zu behandelnde Gegenstand mit der Behandlungslösung 10 Minuten bis 30 Minuten in Kontakt gebracht wird.

9. Verwendung nach Anspruch 1, wobei der pH-Wert der Behandlungslösung 10 bis 13 ist.

10. Verwendung nach Anspruch 1, wobei die Temperatur der Behandlungslösung 0°C bis 60°C ist.

## Revendications

1. Utilisation d'une solution de traitement contenant un hypohalogénite et / ou un ion d'acide hypohalogéneux dans une concentration d'au moins 0,1 % en poids par volume et inférieure à 1 % en poids par volume pour la désactivation de prions, dans laquelle l'objet destiné à être traité par ladite solution de traitement est un verre de contact.

2. Utilisation selon la revendication 1, dans laquelle la concentration de l'hypohalogénite et / ou de l'ion d'acide hypohalogéneux est de 0,1 à 0,7 % en poids par volume.

3. Utilisation selon la revendication 1 ou 2, dans laquelle le verre de contact est mis en contact avec ladite solution de traitement.

4. Utilisation selon la revendication 1, dans laquelle l'hypohalogénite est un hypochlorite ou un hypobromite.

5. Utilisation selon la revendication 1, dans laquelle l'ion d'acide hypohalogéneux est un ion d'acide hypochloreux ou un ion d'acide hypobromeux.

6. Utilisation selon la revendication 1, dans laquelle l'objet destiné à être traité est mis en contact avec la solution de traitement pendant une durée de 3 minutes à 1 heure.

7. Utilisation selon la revendication 1, dans laquelle l'objet destiné à être traité est mis en contact avec la solution de traitement pendant une durée de 5 à 50 minutes.

8. Utilisation selon la revendication 1, dans laquelle l'objet destiné à être traité est mis en contact avec la solution de traitement pendant une durée de 10 à 30 minutes.

9. Utilisation selon la revendication 1, dans laquelle le pH de la solution de traitement est de 10 à 13.

10. Utilisation selon la revendication 1, dans laquelle la température de la solution de traitement est de 0 °C à 60 °C.
